(19) Europäisches Patentamt
European Patent Office
Office européen des brevets

(11) **EP 2 409 874 A1**

(12) **EUROPÄISCHE PATENTANMELDUNG**

(43) Veröffentlichungstag:
**25.01.2012  Patentblatt 2012/04**

(51) Int Cl.:
**B60K 28/06** (2006.01)

(21) Anmeldenummer: **11172363.1**

(22) Anmeldetag: **01.07.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **21.07.2010  US 366177 P**

(71) Anmelder: **BIOTRONIK SE & Co. KG**
**12359 Berlin (DE)**

(72) Erfinder:
• **Dörr, Thomas**
 **12437 Berlin (DE)**
• **Diebold, Michael**
 **12169 Berlin (DE)**

(74) Vertreter: **Lindner-Vogt, Karin L.**
**Biotronik GmbH & Co. KG**
**Corporate Intellectual Properties**
**Woermannkehre 1**
**12359 Berlin (DE)**

(54) **Bedientauglichkeits-Überwachungssystem**

(57)   Die Erfindung betrifft ein Bedientauglichkeits-Überwachungssystem mit einem Beschleunigungssensor, einer Telemetrieeinheit und einer Auswerteeinheit, von denen der Beschleunigungssensor und die Telemetrieeinheit von einem Maschinenführer zu tragen oder implantierbar ausgebildet und mit der Auswerteeinheit verbunden sind und von denen die Auswerteeinheit ausgebildet ist, von dem personengebundenen Beschleunigungssensor stammende Beschleunigungswerte und von einem weiteren Sensor stammende, Bewegungen und/oder Beschleunigungen eines Fahrzeugs oder bewegten Maschine widerspiegelnden Bewegungswerte entgegenzunehmen und durch Vergleich der Beschleunigungswerte mit den Bewegungswerten auszuwerten.

FIG. 4

EP 2 409 874 A1

**Beschreibung**

Die Erfindung betrifft ein Bedientauglichkeits-Überwachungssystem zum Überwachen der Bedientauglichkeit einer Fahrzeug- oder Maschinenführers zum Bedienen eines Fahrzeugs oder dergleichen, nämlich der Tauglichkeit des Maschinenführers zum Bedienen einer bewegten Maschine, bei der der Maschinenführer seinen Platz auf oder in der Maschine hat, so dass er mit der Maschine mitbewegt wird und infolge der Maschinenbewegung Beschleunigungen ausgesetzt ist.

Der Fahrer eines Fahrzeugs als bewegter Maschine sollte nach Möglichkeit fahrtauglich - oder allgemeiner gesprochen: bedientauglich - sein, um das Fahrzeug ohne Gefahr für sich selbst oder Dritte führen zu können. Es kann vorkommen, dass der Fahrer eines Fahrzeugs genau wie auch eine Bedienperson einer Maschine seine Bedientauglichkeit verliert, z.B. durch einen Schwächeanfall, Müdigkeit oder andere Ursachen.

Die der Erfindung zugrunde liegende Aufgabe ist es, ein Bedientauglichkeits-Überwachungssystem zu schaffen, das Zustände eingeschränkter Bedientauglichkeit nach Möglichkeit erkennt. Beispielsweise soll das Bedientauglichkeits-Überwachungssystem ermöglichen, ein Fahrassistenzsystem in die Lage zu versetzen, den Fahrtauglichkeitszustand eines Patienten herzuleiten und diesen für die Fahrzeugsteuerung berücksichtigen zu können.

Bislang beschriebenen Lösungen zur Fahrzeugbeeinflussung durch elektronische Implantate beschränken sich auf die Auswertung rhythmologischer oder ähnlicher physiologischer Parameter und einer daraus abgeleiteten direkten Fahrzeugbeeinflussung. Häufig ist jedoch die Genese einer vorübergehenden Fahrtauglichkeitsstörung unbekannt und kann dann nicht sicher durch die das für den Patienten indizierte Implantat erkannt werden. Ein typisches Beispiel sind implantierbare EKG-Rekorder, die zur Synkopendiagnostik eingesetzt werden. Diese könnten zwar anhand einer Herzrhythmus-Analyse einen Zustand der Fahruntauglichkeit erkennen. Es ist jedoch bekannt, dass mehr als die Hälfte der Synkopen nicht rhythmusbedingt sind [K. Seidl et al.: Initial experience with implantable loop recorders, Europace, Vol. 2, July 2000], d.h. andere Ursachen haben und damit auch nicht durch o.g. Implantate erkannt werden können.

Erfindungsgemäß wird diese Aufgabe durch ein Bedientauglichkeits-Überwachungssystem mit einem Beschleunigungssensor, einer Telemetrieeinheit und einer Auswerteeinheit gelöst, von denen der Beschleunigungssensor und die Telemetrieeinheit von einem Maschinenführer zu tragen und/oder implantierbar ausgebildet - also personengebunden - und mit der Auswerteeinheit verbunden sind und von denen die Auswerteeinheit ausgebildet ist, von dem zu tragenden und/oder implantierbaren Beschleunigungssensor stammende Beschleunigungswerte und von einem weiteren Sensor stammende, Bewegungen und/oder Beschleunigungen eines Fahrzeugs oder bewegten Maschine widerspiegelnden Bewegungswerte entgegenzunehmen und durch Vergleich der Beschleunigungswerte mit den Bewegungswerten auszuwerten.

Die Beschleunigungswerte und die Bewegungswerte liegen vorzugsweise in Form von Zeitreihen oder Vektoren vor, die jeweils eine Folge von über die Zeit abgetasteten Beschleunigungs- oder Bewegungswerten repräsentieren.

Das erfindungsgemäße Bedientauglichkeits-Überwachungssystem ist somit in der Lage zu erfassen, wie ein Fahrzeug- oder Maschinenführer auf Bewegungen des Fahrzeugs oder der Maschine reagiert und kann daraus einen Bedientauglichkeitszustand ableiten.

Die Erfindung beruht auf dem Gedanken, elektronischen Implantaten oder vergleichbaren Diagnostikgeräten einen von der eigentlichen Implantatsdiagnostik unabhängigen Parameter zur Fahrtauglichkeitsklassifikation einem Fahrassistenzsystem zugänglich zu machen und so auch über die primäre Implantatsdiagnostik hinaus eine generelle Aussage zur Fahrtauglichkeit zu ermöglichen.

Der Erfindung schließt außerdem die Erkenntnis ein, dass ein Fahrzeugführer oder Maschinenführer aktiv die während der Fahrt auftretenden Längs- und Querbeschleunigungen mit seiner Körpermuskulatur ausgleicht, sofern dieser bei Bewusstsein ist. Ist diese typische Ausgleichsbewegung nicht mehr vorhanden, so kann von einer eingeschränkten Fahrtauglichkeit ausgegangen werden.

In einer speziellen Variante des Bedientauglichkeits-Überwachungssystems ergibt sich ein System zur Kommunikation zwischen einem elektronischen medizinischem Gerät und einem Fahrzeugsystem*, mit

- einem persönlichen elektronischen medizinischen Gerät mit einer drahtlosen Telemetrieeinheit und
- einer Telemetrieeinheit in einem Fahrzeug zur direkten oder indirekten Kommunikation mit dem medizinischen Gerät.

Hierbei enthält das medizinische Gerät mindestens einen Beschleunigungssensor, der zwei oder dreidimensionale Beschleunigungswerte des Körpers des Patienten erfasst und diese einer Auswerteeinheit zur Verfügung stellt, wobei die Auswerteeinheit, die Beschleunigungsdaten des medizinischen Gerätes mit den Beschleunigungsdaten und Fahrparametern des Fahrzeugs vergleicht und anhand dieses Vergleiches eine Klassifikation des momentanen Fahrtauglichkeitszustandes des Fahrers ableitet und dies dem Fahrassistenzsystem des Fahrzeugs als Steuergröße zur Verfügung stellt.

Es wird im Regelfall davon ausgegangen, dass im jeweiligen Fahrzeug bzw. einer zu führenden Maschine Sensoren für die Erfassung der Fahrzeugbeschleunigungen, Gierraten etc. - also der Bewegung der Maschine - vorhanden sind.

Gemäß einer bevorzugten Ausführungsvariante sind der Beschleunigungssensor, die Telemetrieeinheit und die Auswerteeinheit Bestandteile eines medizinischen Gerätes. Die Telemetrieeinheit ist dann ausgebildet, Bewegungswerte zu empfangen, die von einem Maschinenüberwachungssystem stammen, und an die Auswerteeinheit weiterzugeben. Vorzugsweise umfasst das Bedientauglichkeits-Überwachungssystem eine Relaisstation für die drahtlose Kommunikation zwischen einem Maschinenüberwachungssystem und der Telemetrieeinheit des medizinischen Gerätes. Die dadurch ermöglichte Kommunikation zwischen dem medizinischem Gerät und dem Fahrzeug über eine zwischengeschaltete Relaisstation, die eine Trennung zwischen medizinischem Gerät und Fahrzeugsystem bewirkt, stellt auch im Fehlerfall sicher, dass eine Funktion des medizinischen Gerätes rückwirkungsfrei vom Fahrzeugsystem ist.

Im letztgenannten Fall kann die Auswerteeinheit auch Bestandteil der Relaisstation sein und empfängt die Beschleunigungswerte drahtlos von dem personengebundenen Beschleunigungssensor und der mit dieser verbundenen Telemetrieeinheit. Die Bewegungswerte, die die Bewegung oder Beschleunigung der Maschine repräsentieren, empfängt die Relaisstation in diesem Fall von dem Maschinenüberwachungssystem.

Gemäß einer dritten Alternative ist die Auswerteeinheit Bestandteil des Maschinenüberwachungssystems.

Die Auswerteeinheit ist vorzugsweise dazu ausgebildet, die Beschleunigungswerte mit den Bewegungswerten zu vergleichen und anhand dieses Vergleiches eine Klassifikation eines momentanen Bedientauglichkeitszustandes abzuleiten und ein Ergebnis der Klassifikation dem Maschinenüberwachungssystem als Steuergröße zur Verfügung zu stellen.

Gemäß einer besonders bevorzugten Ausführungsvariante der Auswerteeinheit ist diese dazu ausgebildet den Vergleich als Parametervergleich der Beschleunigungswerte und der Bewegungswerte auf dem Wege der Kreuzkorrelation, Varianzanalyse oder Analyse der Covarianz durchzuführen.

Zusätzlich oder alternativ kann die Auswerteeinheit auch für den Parametervergleich der Beschleunigungswerte mit den Bewegungswerten ein neuronales Netzwerk aufweisen. Letzteres kann selbstlernend oder mit Bewerter lernend ausgebildet sein.

In jedem Fall ist eine Auswerteeinheit bevorzugt, die dazu ausgebildet ist, eine Analyse für den Parametervergleich zur Fahrtauglichkeitsklassifikation sowohl im Zeit- als auch im Frequenzbereich durchzuführen.

Gemäß einer bevorzugten Ausführungsvariante des Bedientauglichkeits-Überwachungssystems ist dieses dazu ausgebildet, im Falle einer Klassifikation der Bedientauglichkeit als beeinträchtigt, einen Reaktionstest auszulösen. Je nach Ergebnis des Reaktionstests wird die Klassifikation der Bedientauglichkeit durch die Auswerteeinheit bestätigt oder widerrufen.

Das medizinische Gerät ist vorzugsweise ein elektronisches Implantat oder ein anderes, am Körper - vorzugsweise dem Oberkörper - des Patienten getragenes Gerät.

Die Kommunikation zwischen medizinischem Gerät und Maschinensteuerung oder Relaisstation erfolgt vorzugsweise im MICS- oder ISM-Band.

Gemäß einer besonders bevorzugten Ausführungsvariante ist das medizinische Gerät dazu ausgebildet, mindestens einen weiteren physiologischen Parameter zur Bewertung der Bedientauglichkeit an die Auswerteeinheit zu übermitteln.

Eine erfindungsgemäße Lösung der eingangs genannten Aufgabe besteht auch in einer Auswerteeinheit für ein Bedientauglichkeits-Überwachungssystem, die

einem Eingang für Beschleunigungswerte, die die Beschleunigung eines medizinischen Gerätes repräsentieren und einem Eingang für Bewegungswerte, die Bewegungen oder Beschleunigungen einer bewegten Maschine repräsentieren sowie

einem Ausgang für ein Bedientauglichkeitsklassifikationsergebnis aufweist.

Die Auswerteeinheit ist dazu ausgebildet, ein jeweiliges Bedientauglichkeitsklassifikationsergebnis durch Vergleich zeitlich einander zugeordneter Beschleunigungswerte und Bewegungswerte zu bilden.

Vorzugsweise ist die Auswerteeinheit dazu ausgebildet, ein eine eingeschränkte Bedientauglichkeit repräsentierendes Klassifikationsergebnis zu liefern, wenn der Vergleich der Beschleunigungswerten mit den Bewegungswerten ergibt, dass die Beschleunigungswerte und die Bewegungswerte in einem Maß übereinstimmen, das einen vorgegebenen Schwellwert überschreitet. Hierzu kann die Auswerteeinheit ausgebildet sein, den Vergleich der Beschleunigungswerten mit den Bewegungswerten mittels einer Kreuzkorrelation, einer Covarianzanalyse, einer Varianzanalyse und/der eines Mustervergleichs durchzuführen.

Zur Lösung der Aufgabe wird außerdem ein Verfahren zur Klassifikation der Bedientauglichkeit eines Maschinenführers einer bewegten Maschine vorschlagen, dass die folgenden Verfahrensschritte einschließt:

- Zur Verfügung stellen von Beschleunigungswerten, die die Beschleunigung eines medizinischen Gerätes repräsentieren,

- Zur Verfügung stellen von Bewegungswerten, die Bewegungen oder Beschleunigungen einer bewegten Maschine repräsentieren,
- Vergleichen von zeitlich einander zugeordneten Beschleunigungswerten und Bewegungswerten, und
- Klassifizieren der Bedientauglichkeit als eingeschränkt, falls der Vergleich der Beschleunigungswerten mit den Bewegungswerten ergibt, dass die Beschleunigungswerte und die Bewegungswerte in einem Maß übereinstimmen, das einen vorgegebenen Schwellwert überschreitet.

Hierbei kann der Vergleich der zeitlich einander zugeordneten Beschleunigungswerte und Bewegungswerte gemäß bevorzugter Ausführungsvarianten des Verfahrens eine Kreuzkorrelation, eine Covarianzanalyse, eine Varianzanalyse und/der einen Mustervergleich einschließen.

Die Erfindung soll nun anhand von Ausführungsbeispielen mit Bezug auf die Figuren näher erläutert werden. Von den Figuren zeigt:

Fig. 1:   das Blockschaltbild des erfindungsgemäßen Bedientauglichkeitssensors;

Fig.2:   Eine Auswerteeinheit des erfindungsgemäßen Bedientauglichkeits-Überwachungssystems und dessen Schnittstellen;

Fig. 3:   ein Beispiel eines Vergleichs der Bewegungs- und Beschleunigungsinformationen in der Auswerteeinheit;

Fig.4:   ein Ausführungsbeispiel der personengebundenen Komponenten des erfindungsgemäßen Bedientauglichkeits-Überwachungssystems.

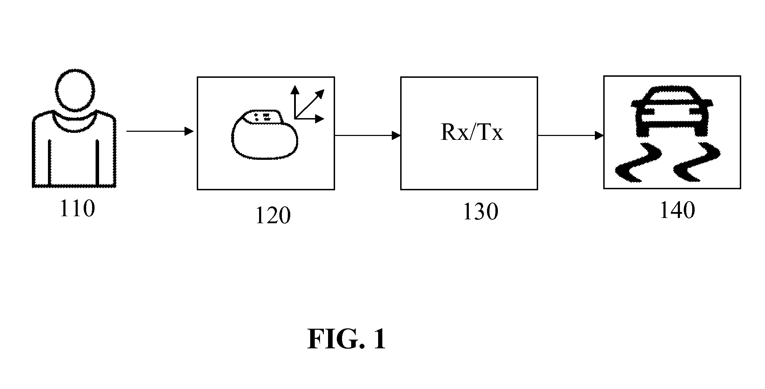

In der Abbildung 1 ist das Blockschaltbild eines erfindungsgemäßen Bedientauglichkeits-Überwachungssystems dargestellt. Der Patient/Fahrer (110) trägt als Maschinenführer ein elektronisches Implantat (120), wie z.B. einen Herzschrittmacher, Defibrillator oder Loop-Rekorder. Dieses elektronische Implantat (120) erfasst mittels eines 3D Beschleunigungssensors permanent die im Oberkörper das Patienten/Fahrers auftretenden Beschleunigungswerte und überträgt diese Information regelmäßig an eine Relaisstation (130) mittels eines MICS-Band-Übertragungsprotokolls. Die Relaisstation (130) wird wiederum regelmäßig von einem Fahrassistenzsystem (140) als Maschinenüberwachungssystem abgefragt. Die so dem Fahrassistenzsystem (140) zur Verfügung gestellten Beschleunigungsdaten werden dann vom Fahrassistenzsystem mit den Beschleunigungsinformationen des Fahrzeugs verglichen und eine Klassifikation der Fahrtauglichkeit daraus abgeleitet.

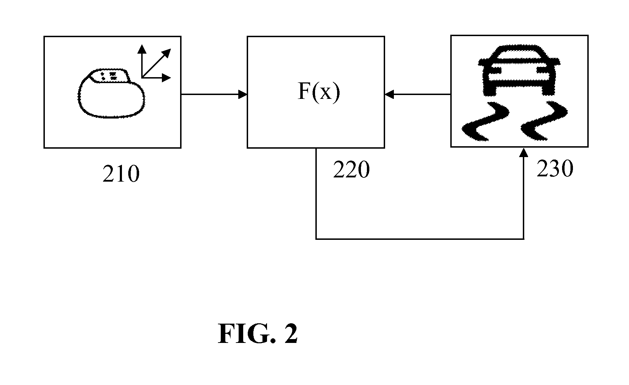

In der Abbildung 2 sind die Auswerteeinheit (220) und ihre Schnittstelen dargestellt. Sowohl das elektronische Implantat als auch das Fahrassistenzsystem senden normierte Beschleunigungsdaten in Form von Beschleunigungsvektoren an die Auswerteeinheit (220). Diese bestimmt dann den Zusammenhang dieser Beschleunigungsvektoren und klassifiziert daraus einen angenommenen Fahrtauglichkeitszustand und signalisiert diesen an das Fahrassistenzsystem (230). Wird eine Einschränkung der Fahrtauglichkeit registriert, so wird zur Plausibilitätsprüfung dem Fahrer/Patienten vom Fahrassistenzsystem ein Reaktionstest abverlangt. So muss der Fahrer z.B. innerhalb einer bestimmten Zeit auf ein optisches, akustisches oder ähnliches Signal adäquat reagieren. Fällt diese Reaktion aus, dann wird die Fahruntauglichkeit vom Fahrassistenzsystem angenommen und eine entsprechende Reaktion eingeleitet. Die Ergebnisse des Reaktionstests können zudem als Bewertungsgröße für eine selbstlernende Auswerteeinheit (220) genutzt werden, z.B. wenn die Auswerteeinheit ein neuronales Netzwerk aufweist, das mit Bewerter selbstlernend ist. Die Ergebnisse des Reaktionstests werden dann dem Bewerter des neuronalen Netzwerks zur Verfügung gestellt.

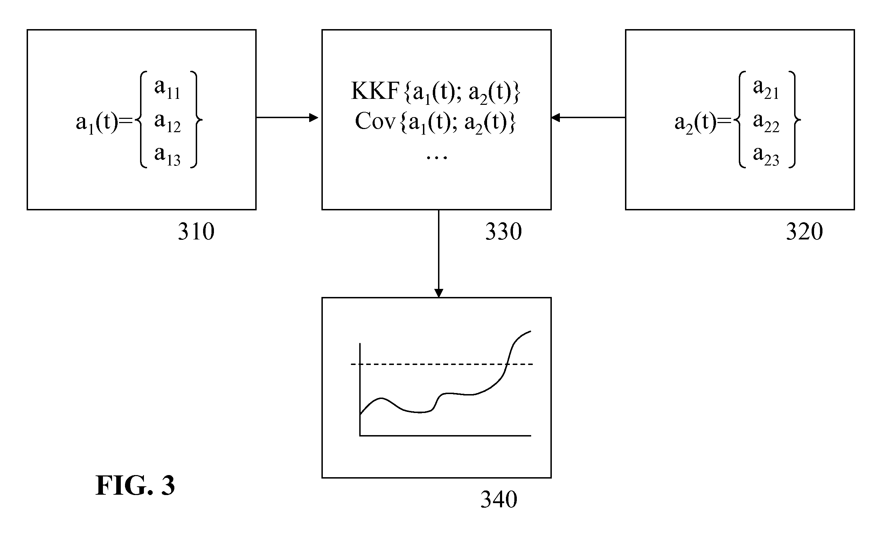

In der Abbildung 3 ist der Vergleich der Beschleunigungsinformationen durch die Auswerteeinheit dargestellt. Als Eingangsgrößen dienen die normierten Beschleunigungsvektoren des medizinischen Gerätes (310) - also die Beschleunigungswerte - und die normierten Beschleunigungsvektoren des Fahrassistenzsystems (320), also die Bewegungswerte. Diese werden mittels mit einer oder mehreren der folgenden Methoden verglichen:

Kreuzkorrelation (KKF)
Covarianzanalyse (Cov)
Varianzanalyse
Mustervergleich (z.B. Pattern matching im neuronalen Netzwerk).

Die Auswerteinheit kann den Vergleich dabei sowohl im Zeit- als auch im Frequenzbereich durchführen.

Überschreitet die Übereinstimmung der beiden verglichenen Vektoren (340) einen Schwellwert, so wird von einen Fahruntauglichkeit ausgegangen, da in diesem Fall die Beschleunigungsdaten des Patienten sich in einer direkten Abhängigkeit von den Fahrzeugbeschleunigungsdaten befinden und damit davon auszugehen ist, dass die normalerweise vorhandene muskuläre Ausgleichsaktivität des Patienten während der Fahrt eingeschränkt ist oder gar nicht stattfindet.

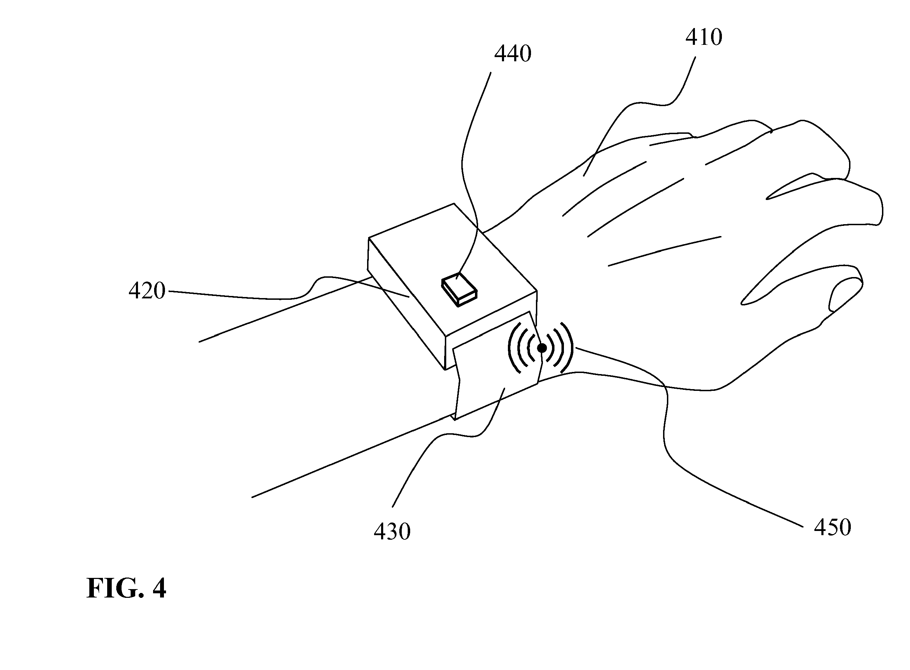

In der Abbildung 4 ist eine spezielle Anordnung des Beschleunigungssensors dargestellt. Hier wird die Beschleunigungsinformation am Handgelenk (410) des Patienten mittels der sog. CardioWatch (420) erfasst. Diese beinhaltet eine 3D-Beschleunigungssensor (440) und wird gleichzeitig als Relaisstation (450) zur RF-Datenübertragung zwischen einem Implantat und dem Fahrzeugsystem eingesetzt. Die für die Telemetrie erforderlichen Antennen sind im Armband (430) der CardioWatch integriert.

Im Folgenden wird eine mögliche Implementierung der Klassifikation der Bedientauglichkeit durch die Auswerteinheit mittels Kreuzkorrelation erläutert.

Als Eingangsgrößen für die Bedientauglichkeitsklassifikation werden der Auswerteeinheit Zeitreihen der bereits normierten Beschleunigungsvektoren des elektronischen Implantates x(t) = {x1(t), x2(t), x3(t)} und Zeitreihen der normierten Beschleunigungsvektoren des Fahrzeuges y(t) = {y1(t), y2(t), y3(t)} verwendet.

Da die Lage bzw. Ausrichtung des im elektronischen Implantat befindlichen Beschleunigungssensors abhängig von der Implantationsposition ist, berechnet die Auswerteinheit zunächst eine grobe Zuordnung zu den Einheitsvektoren des Fahrzeugsystems. Hierfür wird für jede Vektorkombination die Kreuzkorrelationsfunktion nach:

$$R_{xy}(\tau) = \lim_{T_F \to \infty} \frac{1}{T_F} \int_{-T_F/2}^{T_F/2} x(t) \cdot y(t + \tau)\, \mathrm{d}t$$

berechnet, d.h. für:
Rx1y1; Rx1y2; Rx1y3; Rx2y2; Rx2y3 und Rx3y3.

$\tau$ ist dabei unter Berücksichtigung der Abtastrate so gewählt, dass die Kreuzkorrelationsfunktion für mindestens 2 Sekunden breite Fenster bestimmt werden kann, so dass die maximal anzunehmende Phasenverschiebung zwischen den Beschleunigungen des Fahrzeugs und des Implantates/Patienten erfasst wird.

Für die im unmittelbar vorstehend beschrieben Schritt bestimmten Kreuzkorrelationsfunktionen $R_{xy}(\tau)$ ermittelt die Auswerteeinheit nun jeweils das $\tau$ für den Maximalwert bestimmt $\tau_{max}$:

Um das Bewertersignal zur Klassifikation des Fahrtauglichkeitszustandes zu bestimmen, wird der zeitliche Verlauf von $\tau_{max}$ für alle o.g. Korrelationsfunktionen in den Trends aufgezeichnet und analysiert. Die Analyse umfasst dabei vor allem Sprung und Stabilitätsktiterien. Erfolgt z.B. eine plötzliche und anhaltende Veränderung von $\tau_{max}$ in X aus Y Korrelationsfunktionen, so ist der Verdacht einer Fahruntauglichkeit gegeben und wird an das Fahrassistenzsystem gemeldet.

Diese gesamte Berechnung/Bewertung erfolgt regelmäßig, so dass eine vollständige Berechung und Bewertung mindestens alle 0,1s erfolgt.

Tritt ein solcher Verdachtsfall einer Fahrtauglichkeitseinschränkung auf, so wird zunächst der Fahrer zur einem "Reaktionstest" aufgefordert. Erfolgt nicht die angeforderte Reaktion innerhalb einer Zeit von z.B. 2s oder geschwindigkeits- bzw. streckenverlaufsabhängig, so gilt die Fahrtauglichkeit als definitiv eingeschränkt und das Fahrassistenzsystem greift aktiv in den Fahrverlauf ein.

Die erfindungsgemäße Lösung bietet den Vorteil, dass eine Bewertung eines Fahrtauglichkeitszustandes durch ein Fahrassistenzsystem für Träger elektronische medizinischer Geräte/Implantate einfach und robust realisiert werden kann. Der Vorteil der erfindungsgemä-βen Lösung besteht vor allem darin, dass diese Klassifikation unabhängig von der eigentlichen Implantatsdiagnostik erfolgt und so auch Zustände der Fahruntauglichkeit erfasst werden, deren Ursache unabhängig von der Indikation des medizinischen Gerätes sind.

**Patentansprüche**

1. Bedientauglichkeits-Überwachungssystem mit einem Beschleunigungssensor, einer Telemetrieeinheit und einer Auswerteeinheit, von denen der Beschleunigungssensor und die Telemetrieeinheit von einem Maschinenführer zu tragen und/oder implantierbar ausgebildet und mit der Auswerteeinheit verbunden sind und von denen die Auswerteeinheit ausgebildet ist, von dem personengebundenen Beschleunigungssensor stammende Beschleunigungswerte und von einem weiteren Sensor stammende, Bewegungen und/oder Beschleunigungen eines Fahrzeugs oder bewegten Maschine widerspiegelnden Bewegungswerte entgegenzunehmen und durch Vergleich der Beschleunigungswerte mit den Bewegungswerten auszuwerten.

2. Bedientauglichkeits-Überwachungssystem nach Anspruch 1, **dadurch gekennzeichnet, dass** der Beschleunigungssensor, die Telemetrieeinheit und die Auswerteeinheit Bestandteile eines medizinischen Gerätes sind und die Telemetrieeinheit ausgebildet ist, Bewegungswerte zu empfangen, die von einem Maschinenüberwachungssystem stammen.

3. Bedientauglichkeits-Überwachungssystem nach Anspruch 2, **dadurch gekennzeichnet, dass** das Bedientauglichkeits-Überwachungssystem eine Relaisstation für die drahtlose Kommunikation zwischen einem Maschinenüberwachungssystem und der Telemetrieeinheit des medizinischen Gerätes aufweist, die sicherstellt, dass auch im Fehlerfall eine Funktion des medizinischen Gerätes rückwirkungsfrei vom Maschinenüberwachungssystem ist.

4. Bedientauglichkeits-Überwachungssystem nach Anspruch 3, **dadurch gekennzeichnet, dass** die Auswerteinheit Bestandteil der Relaisstation ist und ausgebildet ist, die Beschleunigungswerte drahtlos von dem personengebundenen Beschleunigungssensor und der mit diesem verbundenen Telemetrieeinheit zu empfangen und die Bewegungswerte, die die Bewegung oder Beschleunigung der Maschine repräsentieren, von dem Maschinenüberwachungssystem zu empfangen.

5. Bedientauglichkeits-Überwachungssystem nach einem der Ansprüche 2 bis 3, **dadurch gekennzeichnet, dass** die Auswerteinheit Bestandteil des Maschinenüberwachungssystems ist.

6. Bedientauglichkeits-Überwachungssystem nach einem der Ansprüche 1 bis 5, **dadurch gekennzeichnet, dass** die Auswerteeinheit ausgebildet ist, die Beschleunigungswerte mit den Bewegungswerten zu vergleichen und anhand dieses Vergleiches eine Klassifikation eines momentanen Bedientauglichkeitszustandes abzuleiten und ein Ergebnis der Klassifikation dem Fahrzeugüberwachungssystem als Steuergröße zur Verfügung zu stellen.

7. Bedientauglichkeits-Überwachungssystem nach Anspruch 6, **dadurch gekennzeichnet, dass** die Auswerteinheit dazu ausgebildet ist, den Vergleich als Parametervergleich der Beschleunigungswerte und der Bewegungswerte auf dem Wege der Kreuzkorrelation, Varianzanalyse oder Analyse der Covarianz durchzuführen.

8. Bedientauglichkeits-Überwachungssystem nach Anspruch 6 oder 7, **dadurch gekennzeichnet, dass** die Auswerteinheit ein neuronales Netzwerk für den Parametervergleich der Beschleunigungswerte mit den Bewegungswerten aufweist.

9. Bedientauglichkeits-Überwachungssystem nach einem der Ansprüche 6 bis 8, **dadurch gekennzeichnet, dass** die Auswerteeinheit ausgebildet ist, eine Analyse für den Parametervergleich zur Bedientauglichkeitsklassifikation sowohl im Zeit- als auch im Frequenzbereich durchzuführen.

10. Bedientauglichkeits-Überwachungssystem nach einem der Ansprüche 6 bis 9, **dadurch gekennzeichnet, dass** das Bedientauglichkeits-Überwachungssystems dazu ausgebildet ist, im Falle einer Klassifikation der Bedientauglichkeit als beeinträchtigt einen Reaktionstest auszulösen, wobei die Auswerteeinheit dazu ausgebildet ist die Klassifikation der Bedientauglichkeit je nach Ergebnis des Reaktionstests zu bestätigen oder zu widerrufen.

11. Auswerteeinheit für ein Bedientauglichkeits-Überwachungssystem, mit einem Eingang für Beschleunigungswerte, die die Beschleunigung eines medizinischen Gerätes repräsentieren und
einem Eingang für Bewegungswerte, die Bewegungen oder Beschleunigungen einer bewegten Maschine repräsentieren sowie
einem Ausgang für ein Bedientauglichkeitsklassifikationsergebnis,
wobei die Auswerteeinheit dazu ausgebildet ist, ein jeweiliges Bedientauglichkeitsklassifikationsergebnis durch Vergleich zeitlich einander zugeordneter Beschleunigungswerte und Bewegungswerte zu bilden.

12. Auswerteeinheit nach Anspruch 11, **dadurch gekennzeichnet, dass** die Auswerteinheit dazu ausgebildet ist, ein eine eingeschränkte Bedientauglichkeit repräsentierendes Klassifikationsergebnis zu liefern, wenn der Vergleich der Beschleunigungswerten mit den Bewegungswerten ergibt, dass die Beschleunigungswerte und die Bewegungswerte in einem Maß übereinstimmen, das einen vorgegebenen Schwellwert überschreitet.

13. Auswerteeinheit ach Anspruch 11 oder 12, **dadurch gekennzeichnet, dass** die Auswerteinheit ausgebildet ist, den Vergleich der Beschleunigungswerten mit den Bewegungswerten mittels einer Kreuzkorrelation, einer Covarianzanalyse, einer Varianzanalyse und/der eines Mustervergleichs durchzuführen.

14. Verfahren zur Klassifikation der Bedientauglichkeit eines Maschinenführers einer bewegten Maschine mit den Verfahrensschritten:

   - Zur Verfügung stellen von Beschleunigungswerten, die die Beschleunigung eines medizinischen Gerätes repräsentieren,
   - Zur Verfügung stellen von Bewegungswerten, die Bewegungen oder Beschleunigungen einer bewegten Maschine repräsentieren,
   - Vergleichen von zeitlich einander zugeordneten Beschleunigungswerten und Bewegungswerten, und
   - Klassifizieren der Bedientauglichkeit als eingeschränkt, falls der Vergleich der Beschleunigungswerten mit den Bewegungswerten ergibt, dass die Beschleunigungswerte und die Bewegungswerte in einem Maß übereinstimmen, das einen vorgegebenen Schwellwert überschreitet.

15. Verfahren nach Anspruch 14, **dadurch gekennzeichnet, dass** der Vergleich der zeitlich einander zugeordneten Beschleunigungswerte und Bewegungswerte eine Kreuzkorrelation, eine Covarianzanalyse, eine Varianzanalyse und/der einen Mustervergleich einschließt.

110 120 130 140

**FIG. 1**

EP 2 409 874 A1

**FIG. 2**

$$a_1(t) = \begin{cases} a_{11} \\ a_{12} \\ a_{13} \end{cases}$$

310

$$a_2(t) = \begin{cases} a_{21} \\ a_{22} \\ a_{23} \end{cases}$$

320

KKF $\{a_1(t); a_2(t)\}$
Cov $\{a_1(t); a_2(t)\}$
. . .

330

340

**FIG. 3**

**FIG. 4**

EP 2 409 874 A1

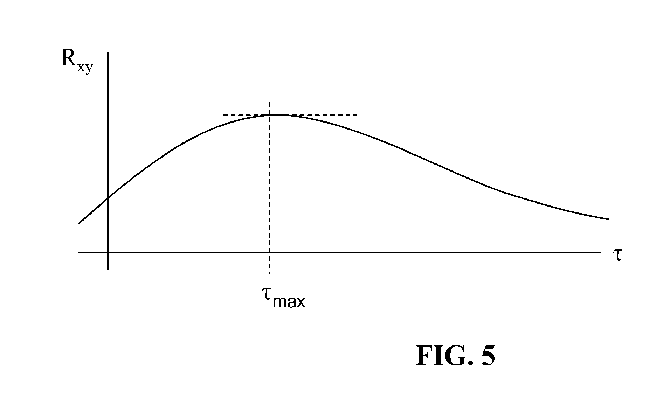

FIG. 5

EP 2 409 874 A1

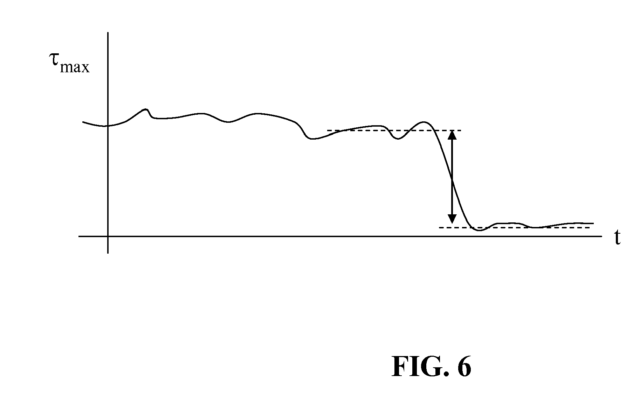

**FIG. 6**

**EP 2 409 874 A1**

Europäisches Patentamt
European Patent Office
Office européen des brevets

# EUROPÄISCHER RECHERCHENBERICHT

Nummer der Anmeldung

EP 11 17 2363

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 661 511 A1 (MATSUSHITA ELECTRIC IND CO LTD [JP]) 31. Mai 2006 (2006-05-31) | 1,5-7, 9-11,14 | INV. B60K28/06 |
| Y | * das ganze Dokument * ----- | 2-4,8 | |
| Y | US 2004/044293 A1 (BURTON DAVID [AU]) 4. März 2004 (2004-03-04) * das ganze Dokument * ----- | 2-4 | |
| Y | US 6 070 098 A (MOORE-EDE MARTIN C [US] ET AL) 30. Mai 2000 (2000-05-30) * das ganze Dokument * ----- | 8 | |
| A | US 2005/137753 A1 (BASSON SARA H [US] ET AL) 23. Juni 2005 (2005-06-23) * das ganze Dokument * ----- | 1-4 | |

|  |
|---|
| RECHERCHIERTE SACHGEBIETE (IPC) |
| B60K |

Der vorliegende Recherchenbericht wurde für alle Patentansprüche erstellt

| Recherchenort | Abschlußdatum der Recherche | Prüfer |
|---|---|---|
| München | 21. November 2011 | Brachmann, Patrick |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

...................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 11 17 2363

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-11-2011

| Im Recherchenbericht angeführtes Patentdokument | Datum der Veröffentlichung | Mitglied(er) der Patentfamilie | Datum der Veröffentlichung |
|---|---|---|---|
| EP 1661511 A1 | 31-05-2006 | EP 1661511 A1<br>US 2006155175 A1<br>WO 2005023105 A1 | 31-05-2006<br>13-07-2006<br>17-03-2005 |
| US 2004044293 A1 | 04-03-2004 | KEINE | |
| US 6070098 A | 30-05-2000 | US 6070098 A<br>US 6511424 B1 | 30-05-2000<br>28-01-2003 |
| US 2005137753 A1 | 23-06-2005 | US 2005137753 A1<br>US 2007241915 A1 | 23-06-2005<br>18-10-2007 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **K. SEIDL et al.** *Initial experience with implantable loop recorders, Europace,* Juli 2000, vol. 2